(19) Europäisches Patentamt
European Patent Office
Office européen des brevets

(11) Publication number: **0 239 062 B1**

(12) # EUROPEAN PATENT SPECIFICATION

(45) Date of publication of patent specification: **29.05.91 Bulletin 91/22**

(51) Int. Cl.[5]: **C07C 231/00**, C07C 233/47

(21) Application number: **87104291.7**

(22) Date of filing: **24.03.87**

(54) **N2-(1-carboxy-3-oxo-3-phenylpropyl)-L-Lysine compounds and their derivatives.**

(30) Priority: **27.03.86 JP 68970/86**

(43) Date of publication of application: **30.09.87 Bulletin 87/40**

(45) Publication of the grant of the patent: **29.05.91 Bulletin 91/22**

(84) Designated Contracting States: **BE CH DE ES FR GB IT LI NL SE**

(56) References cited:
**EP-A- 0 150 263**
**US-A- 4 626 545**
**CHEMICAL ABSTRACTS, vol. 89, no. 25, 18th December 1978, page 630, column 2, abstract no. 215732p, Columbus, Ohio, US; AGBALYAN et al.: "Addition of aliphatic amino acids to beta-aroyl acrylic acids", ARM. KHIM. ZH. 1978, 31(4), 273-275**
**CHEMICAL ABSTRACTS, vol. 107, no. 7, 17th August 1987, page 781, column 1, abstract no. 59477s, Columbus, Ohio, US.**

(73) Proprietor: **KANEGAFUCHI KAGAKU KOGYO KABUSHIKI KAISHA
2-4 Nakanoshima 3-chome
Kita-ku Osaka-shi Osaka-fu (JP)**

(72) Inventor: **Yamada, Kazuhiko
248-3, Eigashima Okubo-cho
Akashi-shi Hyogo-ken (JP)**
Inventor: **Inoue, Kenji
1-3-31, Asahigaoka Tarumi-ku
Kobe-shi Hyogo-ken (JP)**
Inventor: **Takahashi, Satomi
1-13-13, Shinwadai, Tarumi-ku
Kobe-shi Hyogo-ken (JP)**
Inventor: **Ohashi, Takehisa
9-14, Shinoharaobanoyama-cho 3-chome
Nada-ku
Kobe-shi Hyogo-ken (JP)**
Inventor: **Watanabe, Kiyoshi
15-41, Matsugaoka 5-chome
Akashi-shi Hyogo-ken (JP)**

(74) Representative: **Türk, Gille, Hrabal
Brucknerstrasse 20
W-4000 Düsseldorf 13 (DE)**

EP 0 239 062 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a process for efficiently preparing an $N^2$-(1-carboxy-3-phenylpropyl)-L-lysine derivative, especially an optically active $N^2$-[1-(S)-carboxy-3-phenylpropyl]-L-lysine derivative having the formula (XI) :

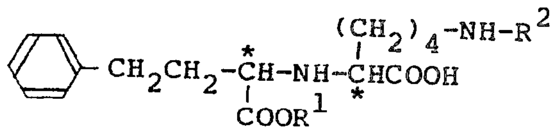

(XI)

wherein $R^1$ is an alkyl group, $R^2$ is an acyl or urethane type protecting group which is stable on catalytic hydrogenolysis and an asterisk (*) represents (S)-configuration with respect to the asymmetric carbon atom.

The present invention aims at preparing in an industrially advantageous manner, an $N^2$-[1-(S)-carboxy-3-phenylpropyl]-L-lysine derivative which is a useful intermediate for preparing $N^2$-[1-(S)-carboxy-3-phenylpropyl]-L-lysyl-L-proline having the formula (V) :

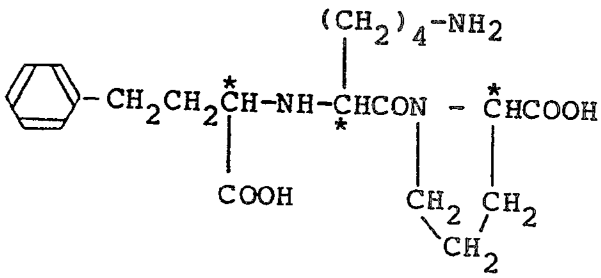

(V)

wherein an asterisk (*) represents (S)-configuration with respect to the asymmetric carbon atom, which is expected to be used as an antihypertensive agent because the proline has an excellent Angiotensin Converting Enzyme (ACE) inhibitory activity.

As a process for preparing $N^2$-(1-carboxy-3-phenylpropyl)-L-lysine derivative, there has been known a method wherein ethyl-β-benzoylacrylate (VI) is reacted with L-lysine ester derivative, $N^6$-benzyloxy-carbonyl-L-lysine benzyl ester (VII), by the so-called Michael addition reaction in the presence of a catalytic amount of triethylamine to give a diastereomeric mixture of $N^2$-(1-ethoxycarbonyl-3-oxo-3-phenylpropyl)-$N^6$- benzyloxycarbonyl-L-lysine benzyl ester (VIII), from which $N^2$-(1-(S)-ethoxycarbonyl-3-oxo-3-phenylpropyl)-$N^6$-benzyloxycarbonyl-L-lysine benzyl ester (VIII-a) having the desired configuration is obtained by a crystallization procedure, the ester (VIII-a) being subjected to catalytic reduction with a catalyst of palladium/carbon to give $N^2$-(1-(S)-ethoxycarbonyl-3-phenylpropyl)-L-lysine (IX), followed by the reaction with chloroformic acid benzyl ester in order to protect amino group at the side chain of lysine and the purification of the product by a silica-gel chromatography to give $N^2$-(1-(S)-ethoxycarbonyl-3-phenylpropyl)-$N^6$-benzyloxycarbonyl-L-lysine (X), as shown in the following reaction scheme (Japanese Unexamined Patent Publication No. 103364/1983) :

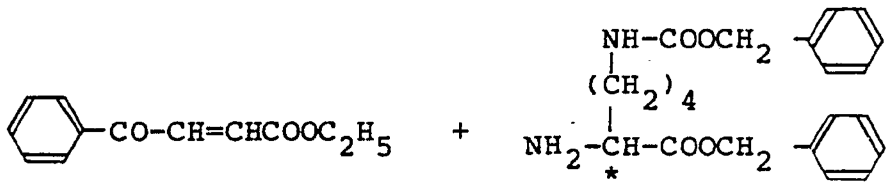

(VI) (VII)

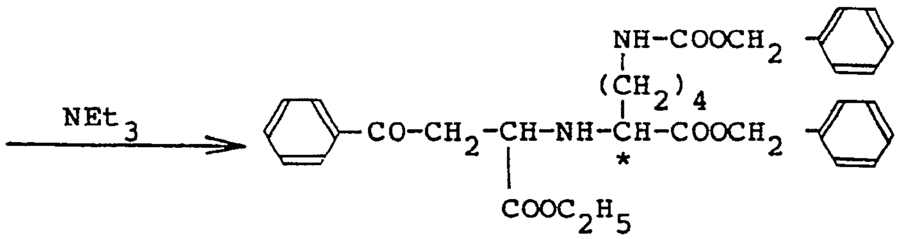


(S, S)----(VIII-a)
(R, S)----(VIII-b)

(VIII-a)

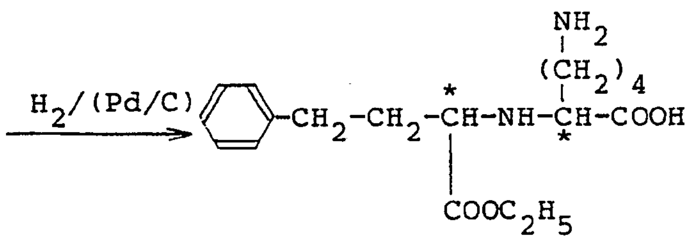


(IX)

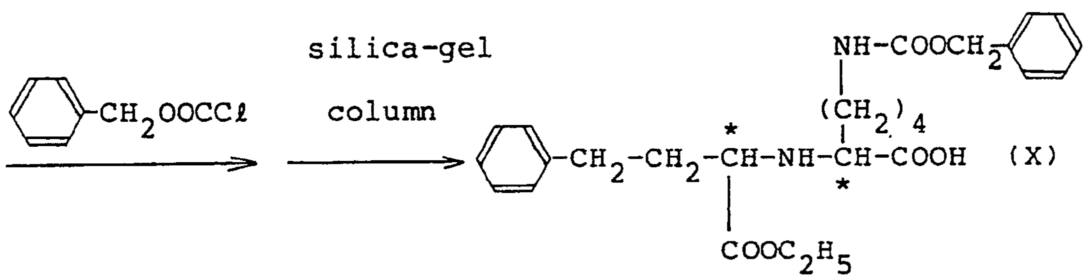


(X)

However, the above method employing L-lysine ester derivative requires a procedure to convert the α-amino group moiety of N[6]-benzyloxycarbonyl-L-lysine benzyl ester, which is present in the form of a salt with an acid used in the esterification, into free amino group in addition to a procedure to esterify L-lysine derivative. Further, it is required to employ such an ester that the ester moiety is selectively converted into carboxylic group without converting ethyl ester moiety of the product derived from ethyl β-benzoylacrylate into carboxylic group. Thus the ester which can be employed in the reaction is limited to the ester such as benzyl ester or tert-butyl ester, which are prepared by relatively complicated procedure. Since lysine is a basic amino acid having amino group at the side chain, it is preferred that the amino group at ε-position is protected by the protective group usually employed in the peptide synthesis in order to carry out the reaction of the amino group only at α-position. When benzyloxycarbonyl group is employed as the protective group, the amino group at ε-position is deprotected by hydrogenolysis in the reduction of the compound (VIII-a) to the compound (IX). In order to use the compound (IX) in the subsequent reaction, the amino group at ε-position in the lysine moiety is preferably protected and thus the compound (IX) is reacted with chloroformic acid benzyl ester to introduce benzyloxycarbonyl

group. However, since the amino group at α-position also has the reactivity as well as the amino group at ε-position in the lysine moiety in the compound (IX), the production of by-product inevitably occurs. Therefore, a molar yield of the compound (X) purified by a silica-gel chromatography is as low as 42% based on the compound (IX) and also a total molar yield is only 15.8% based on starting $N^6$-benzyloxycarbonyl-L-lysine benzyl ester. As mentioned above, the method gives a quite low yield with the employment of the starting materials which are prepared in many steps and has a difficulty in the production of $N^2$-(1-carboxy-3-phenylpropyl)-L-lysine derivative in industrial scale from the viewpoint of operatability and economy.

The present inventors have filed patent applications on a method for economically and efficiently preparing N-[1-(S)-ethoxycarbonyl-3-oxo-3-phenylpropyl]-L-alanine and N-[1-(S)-ethoxycarbonyl-3-phenylpropyl]-L-alanine which are useful intermediates for preparing various Angiotensin Converting Enzyme inhibitory agent (ACEI) (Japanese Patent Application No. 19483/1985), on an inorganic acid salt of N-[1-(S)-ethoxycarbonyl-3-phenylpropyl]-L-alanylchloride which is a reactive derivative efficiently utilized in the synthesis of various ACEI, and a process for efficiently preparing the same (Japanese Patent Application No. 188242/1985), and on a method for economically and efficiently preparing N-[1-(S)-ethoxycarbonyl-3-phenylpropyl]-L-alanyl-L-proline (enalapril), which is one of an ACEI and is expected to have an excellent effect, by using N-[1-(S)-ethoxycarbonyl-3-phenylpropyl]-L-alanine (Japanese Patent Application No. 188243/1985).

An object of the present invention is to provide a simple, economical and efficient process for the industrial production of a $N^2$-[1-(S)-carboxy-3-oxo-3-phenylpropyl]-L-lysine derivative and a $N^2$-[1-(S)-carboxy-3-phenylpropyl]-L-lysine derivative, which are quite useful intermediates for preparing $N^2$-[1-(S)-carboxy-3-phenylpropyl]-L-lysyl-L-proline (lysinopril) expected to be used as an ACEI having extremely excellent effects.

The above and other objects of the present invention will become apparent from the description hereinafter.

As a result of the inventor's continuous studies with above-mentioned technical background, it has now been found that an $N^2$-[1-carboxy-3-oxo-3-phenylpropyl]-L-lysine derivative having the formula (I) :

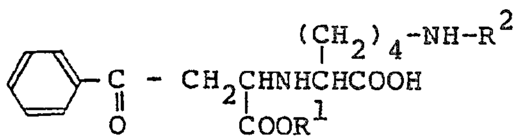

(I)

wherein $R^1$ is an alkyl group and $R^2$ is an acyl or urethane type protecting group, can be obtained in extremely high yield by reacting a β-benzoylacrylic acid ester having the formula (II) :

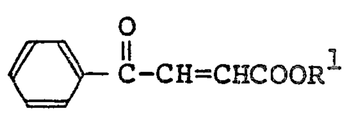

(II)

wherein $R^1$ is an alkyl group, with an L-lysine derivative having the formula (III) :

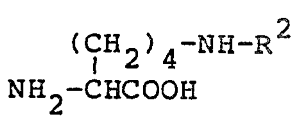

(III)

wherein $R^2$ is an acyl or urethane type protecting group which is stable on catalytic hydrogenolysis in the presence of a base in an amount equivalent to the compound (III), that the desired compound with (S)-configuration with respect to the asymmetric carbon atom can be obtained in a large amount compared with the undesired compound with (R)-configuration by conducting the above-mentioned reaction under specific reaction conditions, and that an $N^2$-(1-carboxy-3-phenylpropyl)-L-lysine derivative having the formula (IV) :

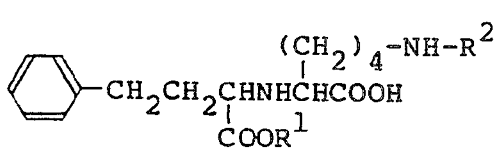

(IV)

wherein $R^1$ and $R^2$ are as defined above, can be easily prepared by conducting catalytic hydrogenolysis of the

lysine derivatives (I).

According to the present invention, there is provided a process for preparing an $N^2$-(1-carboxy-3-oxo-3-phenylpropyl)-L-lysine derivative having the formula (I) :

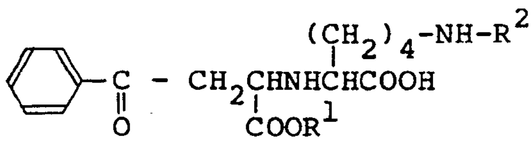

(I)

wherein $R^1$ is an alkyl group and $R^2$ is an acyl or urethane type protecting group, which comprises reacting a β-benzoylacrylic acid ester having the formula (II) :

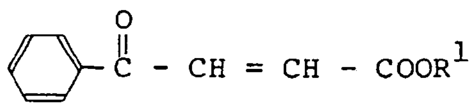

(II)

wherein $R^1$ is as defined above, with an L-lysine derivative having the formula (III) :

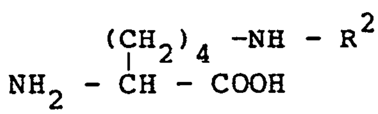

(III)

wherein $R^2$ is as defined above, in the presence of a base in an amount equivalent to the compound (III) and a process for preparing an $N^2$-(1-carboxy-3-phenylpropyl)-L-lysine derivative having the formula (IV) :

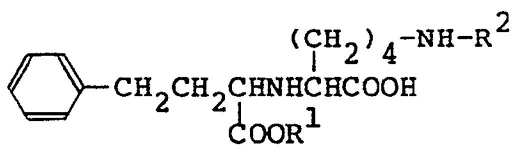

(IV)

wherein $R^1$ and $R^2$ are as defined above, which comprises conducting catalytic hydrogenolysis of an $N^2$-(1-carboxy-3-oxo-3-phenylpropyl)-L-lysine derivative having the formula (I) :

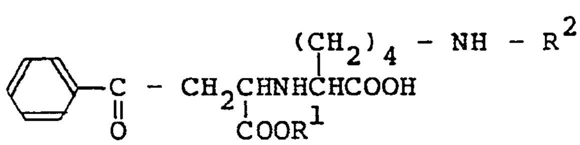

(I)

wherein $R^1$ and $R^2$ are as defined above.

The present invention is shown by the follwoing reaction scheme :

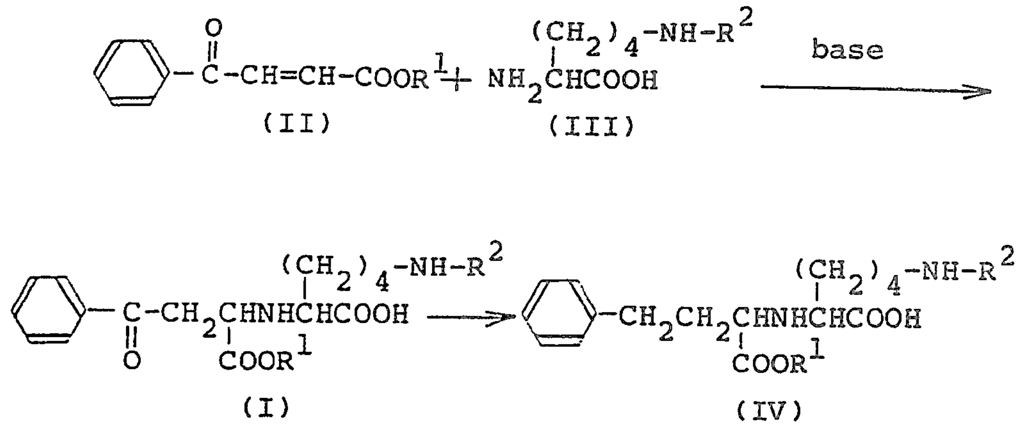


(II) (III)

(I) (IV)

wherein $R^1$ is an alkyl group and $R^2$ is an acyl or urethane type protecting group which is stable on catalytic hydrogenolysis.

As to the β-benzoylacrylic acid ester (II) used as a starting material in the addition reaction of the present invention, there are a trans form and a cis form. The trans-β-benzoylacrylic acid ester can be easily obtained by conducting esterification of a trans-β-benzoylacrylic acid prepared by a conventional method such as Friedel-Crafts reaction of benzene and maleic anhydride or dehydrating condensation of glyoxylic acid and acetophenone. Also, the cis-β-benzoylacrylic acid ester can be prepared by isomerization of the trans-β-benzoylacrylic acid ester with irradiation with light. Both the trans isomer and the cis isomer can be used in the present invention, and it is preferable to use the trans isomer which are prepared by a few steps from the point of industrial use.

Examples of the alkyl group are, for instance, methyl group, ethyl group, n-propyl group, iso-propyl group, n-butyl group, tert-butyl group and the like. The above groups having carbon atoms of from 1 to 4 are preferable since they are stable on catalytic hydrogenolysis. To the contrary, such a group as to be removed on catalylic hydrogenolysis, e.g. benzyl group, nitrobenzyl group or methoxybenzyl group, is not preferable.

The L-lysine derivatives (III) which are other starting materials used in the addition reaction of the present invention have two amino groups at α-position and ε-position of L-lysine. Accordingly, when the L-lysine derivatives in which amino groups are not protected are used in the addition reaction, although the reaction can proceed, there is produced much by-product in which amino group at ε-position of the L-lysine is reacted with the β-benzoylacrylic acid ester besides the desired compound in which amino group at α-position of L-lysine is added, and a ratio of the desired compound to the obtained product is about 50%. Therefore, in order to react the only amino group at α-position with the β-benzoylacrylic acid ester, it is preferable to protect amino group at ε-position with a protecting group usually used in peptide synthesis, but which is stable on catalytic hydrogenolysis. Examples of such protecting group are, for instance, an urethane type protecting group such as tertiary butyloxycarbonyl (Boc) group, methylsulfonylethyloxycarbonyl (Msoc) group, amyloxycarbonyl (Acc) group or isobornyloxycarbonyl (Iboc) group, an acyl type protecting group such as trifluoroacetyl group, formyl group or phthaloyl group and the like.

Since a lysine with the protected amino group at ε-position, which exists in a state of ampho-ion as in other neutral amino acids, has a protonized amino group at a-position, the lysine has no nucleophilicity and does not show reactivity on a β-benzoylacrylic acid. In case of neutral amino acid present in a state of ampho-ion, it is generally known that the ionization state varies with pH of the solution as follows :

$$\overset{+}{H_3N}\text{-CH(R)-COOH} \underset{+H^+}{\overset{-H^+(pK'a_1)}{\rightleftharpoons}} \overset{+}{H_3N}\text{-CH(R)-COO}^- \underset{+H^+}{\overset{-H^+(pK'a_2)}{\rightleftharpoons}}$$

$$H_2N\text{-CH(R)-COO}^-$$

wherein R represents the side chain of amino acid.

The addition of a base anionizes a lysine with the protected amino group at ε-position, resulting in the lysine

serving as an amine component. Therefore, it is presumed that a salt formed from the lysine with the protected amino group at ε-position and the base can substantially be subjected to the reaction. Preferably, the base is used in a stoichiochemically equivalent amount to the lysine with the protected amino group at ε-position.

Examples of the base which can be employed in the reaction are an alkali metal hydroxide, an alkaline earth metal hydroxide, an alkali metal carbonate, an quarternary ammonium hydroxide, ammonia and an amine. Examples of quarternary ammonium hydroxide are, for instance, a hydroxide of tetramethyl ammonium, tetraethyl ammonium, tetrapropyl ammonium, tetrabutyl ammonium, tetraamyl ammonium, tetrahexyl ammonium, tetraoctyl ammonium, benzyltrimethyl ammonium, benzyltriethyl ammonium, cetyltrimethyl ammonium, decyltrimethyl ammonium, ethyltrimethyl ammonium, octyltrimethyl ammonium, phenyltrimethyl ammonium, trimethylstearyl ammonium, β-hydroxyethyltrimethyl ammonium, trioctylmethyl ammonium, tetradecyldimethylbenzyl ammonium and the like. Examples of amine are, for instance, a primary amine such as methylamine, ethylamine, propylamine, butylamine, amylamine, hexylamine, cyclohexylamine, heptylamine, octylamine, allylamine, α-phenylethylamine or β-phenylethylamine ; a secondary amine such as dimethylamine, diethylamine, dipropylamine, dibutylamine, diamylamine, dehexylamine, dicyclohexylamine, diallylamine, morpholine, piperidine or hexamethyleneimine and a tertiary amine such as trimethylamine, triethylamine, tripropylamine, tributylamine, triamylamine, trihexylamine, triallylamine triethanolamine, N-methylmorpholine, N,N-dimethylcyclohexylamine, N,N,N′,N″-tetramethylethylenediamine or 4-dimethylaminopyridine.

Examples of such alkali metal hydroxide are, for instance, lithium hydroxide, sodium hydroxide, potassium hydroxide and the like. Examples of alkaline earth metal hydroxide are, for instance, magnesium hydroxide, calcium hydroxide, barium hydroxide and the like. And Examples of such alkali metal carbonate are, for instance, lithium carbonate, sodium carbonate, potassium carbonate, sodium hydrogencarbonate, potassium hydrogencarbonate and the like.

When ε-amino group in the lysine is protected with a group which does not react with the above-metnioned bases, a salt can be easily prepared by a method in which the base and the lysine in which ε-amino group is protected in a solvent such as water or a alcohol are stirred at a room temperature or under heating, and then the salt can be reacted with the benzoylacrylic acid derivative.

It is also possible to carry out the reaction mildly with preparing the salt in situ in the reaction system by adding a stoichimetrically necessary amount of the base to the mixture of β-benzoylacrylic acid ester and the ε-amino group-protected lysine.

As a reaction solvent used in the Michael additon reaction of the β-benzoylacrylic acid derivative and the lysine in which ε-amino group is protected in the presence of a base, there are exemplified, for instance, water, an alcohol such as methanol, ethanol, propanol or butanol, an ether such as dioxane or tetrahydrofuran, n-hexane, acetonitrile, a mixture solvent thereof, and the like. Usually, it is preferable to use a alcohol-water solvent. The addition reaction can proceed rapidly in the alcohol-water solvent, and is usually completed at room temperature for several minutes to 1 hour.

In Michael addition reaction of the β-benzoylacrylic acid derivative and the L-lysine derivative, the reaction condition to be taken so that the production ratio of the desired $N^2$-[1-(S)-carboxy-3-phenylpropyl]-L-lysine derivative having the (S, S)-configuration can be selectively heightened depends on which the β-henzoylacrylic acid derivative, and the lysine derivative are employed. As factors importantly influencing the additon reaction, there are exempiefied the reaction temperature, the pH of the reaction system, kinds of protective groups of ε-amino group in the L-lysine derivative, kinds of the base employed.

The reaction temperature is not particularly limited and the addition raction can be carried out in a wide range of temperature. However, when the $N^2$-(1-carboxy-3-oxo-3-phenylpropyl)-L-lysine derivative produced is allowed to stand under the alkali condition,the product may be decreased with the passage of time or the composition ratio between diastereomers may be changed. Therefore, it is preferable to conduct the addition reaction at a temperature of not more than 25°C, more preferably not more than 15°C in order to maintain the high production ratio of the $N^2$-(1-carboxy-3-oxo-3-phenylpropyl)-L-lysine derivative (I) having the (S, S)-configuration because the composition ratio between diastereomers can be changed during the addition reaction. When after completing the addition reaction, an acid, particularly a mineral acid such as hydrochloric acid or sulfuric acid, is added to the reaction mixture in an amount of more than equivalent of the used alkali, quaternary ammonium or weakly base to acidify the reaction system, the reaction system is stabilized, that is, the change of the composition ratio between diastereomers does not occur, and the later operations can be conducted easily.

The $N^2$-(1-carboxy-3-oxo-3-phenylpropyl)-L-lysine derivative can be easily isolated from the reaction mixture according to usual methods such that the alkali is neutralized with the acid and then the solvent is distilled away from the reaction mixture under reduced pressure. If necessary, it is possible to extract the desired compound from a solvent with a solvent to isolate after neutralizing and distilling away the solvent from the reaction mixture under reduced pressure. Also, the derivative can be isolated as a hydrochloric acid salt or a sulfuric

acid salt. Further, it is possible to reduce the addition reaction mixture as it is without conducting the isolation of the $N^2$-(1-carboxy-3-oxo-3-phenylpropyl)-L-lysine derivative and to isolate the $N^2$-(1-carboxy-3-phenylpropyl)-L-lysine derivative (IV).

For example, when ethyl trans-β-benzoylacrylate is reacted with ε-trifluoroacetyl-L-lysine in ethanol-water, it is suitable to use lithium hydroxide, sodium hydroxide or potassium hydroxide as the alkali metal. Among them, when using the lithium hydroxide, the desired $N^2$-[1-(S)-ethoxycarbonyl-3-oxo-3-phenylpropyl]-$N^6$-trifluoroacetyl-L-lysine can be obtained in remarkably high selectivity, i.e. a ratio of the (S, S)-form/the (R, S)-form is 82/18. Also, the composition ratio between diastereomers is scarcely changed at a temperature of not more than 10°C.

After completing the addition reaction, the mineral acid such as hydrochloric acid or sulfuric acid is rapidly added to the reaction system to convert the alkali salt of $N^2$-(1-ethoxycarbonyl-3-oxo-3-phenylpropyl)-$N^6$-trifluoroacetyl-L-lysine into $N^2$-(1-ethoxycarbonyl-3-oxo-phenylpropyl)-$N^6$-trifluoroacetyl-L-lysine, or the hydrochloride or sulfate thereof, and the desired compound can be stably isolated in a usual manner. Also, it is possible to conduct the reduction reaction continuously without isolating $N^2$-(1-ethoxycarbonyl-3-oxo-3-phenylpropyl)-$N^6$-trifluoroacetyl-L-lysine after adding hydrochloric acid or sulfuric acid in an amount of more than the equivalent of the alkali used in the addition reaction.

The $N^2$-(1-carboxy-3-oxo-3-phenylpropyl)-L-lysine derivative can be subjected to catalytic hydrogenolysis proceeding gently in water, an alcohol or a polar protic solvent such as acetic acid in the presence of a suitable amount of an acid such as sulfuric acid, hydrochloric acid or formic acid to give the $N^2$-(1-carboxy-3-phenylpropyl)-L-lysine derivative in a high yield. In catalytic hydrogenolysis, there are exemplified, for instance, palladium, Raney nickel, and the like as suitable catalysts. For instance, when $N^2$-(1-ethoxycarbonyl-3-oxo-phenylpropyl)-$N^6$-trifluoroacetyl-L-lysine is subjected to the catalytic hydrogenolysis, the lysine derivative is hydrogenated in the alcohol such as ethanol as the solvent in the presence of the acid at a temperature of 0°C to 60°C, preferably from 5°C to 40°C for several to 24 hours by using palladium/carbon as the catalyst to give almost quantitatively $N^2$-(1-ethoxycarbonyl-3-phenylpropyl)-$N^6$-trifluoroacety-L-lysine. After completing the hydrogenation, the catalyst is removed from the reaction mixture, the acid of the resulting g solution is neutralized with an alkali, from which the solvent is distilled away and then the desired compound having the (S, S)-configuration can be isolated in a usual manner such as extraction. If necessary, the recrystallization can be conducted from the obtained reaction mixture.

The thus obtained the $N^2$-(1-carboxy-3-phenylpropyl)-L-lysine derivatives, especially the $N^2$-[1-(S)-carboxy-3-phenylpropyl]-L-lysine derivatives, can be easily converted into the lysinopril derivatives in a known manner applied to peptide synthesis, such as acid chloride method, NCA method, activated ester method or mixed anhydride method.

As aforementioned, in the present invention, there can be economically obtained in a high yield the $N^2$-(1-carboxy-3-oxo-3-pheylpropyl)-L-lysine derivatives, especially the $N^2$-[1-(S)-carboxy-3-oxo-3-phenylpropyl]-L-lysine derivatives being the optically active compounds and the reduced compounds thereof, the derivatives being useful as the intermediates of lysinopril which is expected to be used as an antihypertensive agent, and the present invention provides a remarkably useful method in the economically and efficiently industrial production of lysinopril.

The present invention is more specifically described and explained by the following Examples.

The quantitative analysis was done by a high performance liquid chromatography (HPLC). As aforementioned, $N^2$-(1-carboxy-3-oxo-3-phenylpropyl)-L-lysine derivatives are slightly unstable under an alkaline and are thermodynamically easily changed from the (S, S) form to the (R, S) form. Therefore, the tested samples were analyzed after being enough acidified so as not to change the ratio of the (S, S) form and the (R, S) form. The following conditions were applied to the analysis unless otherwise noted and the (S, S) form and the (R, S) form were separated completely and determined. The ratio of solvent of Mobile phase was optionally adjusted according to the polarity of $N^2$-(1-carboxy-3-oxo-3-phenylpropyl)-L-lysine derivatives.

Column : Finepak SIL $C_{18-5}$ (made by Japan Spectroscopiec Co., Ltd.), 4.6 mm ID × 250 mm
Mobile phase : 60 mM phosphate buffer (pH 2.5)/acetonitrile = 75/25 (v/v)
Flow rate : 1.2 ml/min
Detection : 210 nm
Internal standard : 5-benzyl hydantoin

Examples 1 to 6

There was dissolved 40.8 mg of trans-ethyl β-benzoylacrylicate (hereinafter referred to as "t-EBA") in 2.0 ml of a solution of ethanol and water in a volume ratio of 3 : 1 and then 48.4 mg of $N^6$-trifluoroacetyl-L-lysine (hereinafter referred to as "L-Lys (Tfa)") was added. And to the suspension,0.2 ml of the solution or suspension

containing an alkali metal hydroxide (0.2 milimol) or an alkali earth metal hydroxide (0.1 mmol) shown in Table 1 was added rapidly while cooling with ice at 0°C and was stirred for 30 minutes. After stopping the reaction by adding 0.5 ml of 1N-HCℓ (0.5 mmol) the product was analyzed by HPLC and the formation of $N^2$-(1-ethoxycarbonyl-3-oxo-3-phenylpropyl)-$N^6$-trifluoroacetyl)-L-lysine was found.

The amounts of the obtained (S, S) form and (R, S) form are shown in Table 1.

Reference Example 1

The procedures in Examples 1 to 6 were repeated without any alkali metal hydroxide or alkali earth metal hydroxide.

The amounts of the obtained (S, S) form and (R, S) form are shown in Table 1.

Table 1

| Example No. or Reference Example No. | alkali metal or alkali earth metal | amount of the (S,S) form (mg) | amount of the (R,S) form (mg) |
|---|---|---|---|
| Ex. 1 | Li | 41.1 | 10.6 |
| Ex. 2 | Na | 38.8 | 12,6 |
| Ex. 3 | K | 42.6 | 13.7 |
| Ex. 4 | Mg | 7.1 | 3.8 |
| Ex. 5 | Ca | 42.2 | 25.4 |
| Ex. 6 | Ba | 26.9 | 15.9 |
| Ref.Ex.1 | - | 0 | 0 |

Examples 7 to 42

To a suspension consisting of 204.0 mg of t-EBA, 254.0 mg of L-Lys(Tfa) and 5.0 ml of solution of ethanol and water in a volume ratio of 3 : 1 was added 1.0 mmol of an amine, a quarternary ammonium hydroxide or ammonia shown in Table 2 at once while stirring by a magnetic stirrer at 0°C and the stirring was continued for 60 minutes. Then the reaction was stopped by adding 3 ml of 1N-HCℓ. The obtained product was analyzed by HPLC and the formation of $N^2$-(1-ethoxycarbonyl-3-oxophenylpropyl)-$N^6$-trifluoroacetyl-L-lysine was found.

The amounts of the obtained (S, S) form and (R, S) form are shown in Table 2.

Reference Example 2

The procedures in Examples 7 to 41 were repeated without any amines, quarternary ammonium hydroxides or ammonia.

The amounts of the obtained (S, S) form and (R, S) form are shown in Table 2.

Table 2

| Example No. or Reference Example No. | amine, quarternary ammonium hydroxide or ammonia | amount of the (S,S) form (mg) | amount of the (R,S) form (mg) |
|---|---|---|---|
| Ex. 7 | ethylamine | 46.8 | 19.1 |
| Ex. 8 | n-propylamine | 49.9 | 19.2 |
| Ex. 9 | iso-propylamine | 116.7 | 43.3 |
| Ex.10 | n-butylamine | 58.4 | 22.7 |
| Ex.11 | sec-butylamine | 136.7 | 50.5 |
| Ex.12 | tert-butylamine | 237.9 | 87.5 |
| Ex.13 | n-hexylamine | 25.4 | 10.2 |
| Ex.14 | n-octylamine | 10.8 | 4.3 |
| Ex.15 | diethylamine | 123.0 | 50.5 |
| Ex.16 | di-n-propylamine | 203.5 | 81.1 |
| Ex.17 | di-iso-propylamine | 199.9 | 79.3 |
| Ex.18 | di-n-butylamine | 184.5 | 71.0 |
| Ex.19 | di-iso-butylamine | 153.9 | 61.0 |
| Ex.20 | di-n-hexylamine | 20.8 | 7.7 |
| Ex.21 | dicyclohexylamine | 385.0 | 72.0 |
| Ex.22 | morpholine | 3.4 | 2.3 |
| Ex.23 | piperidine | 92.6 | 28.6 |
| Ex.24 | hexamethyleneimine | 92.5 | 34.0 |

- continued -

- continued -

| Example No. or Reference Example No. | amine, quarternary ammonium hydroxide or ammonia | amount of the (S,S) form (mg) | amount of the (R,S) form (mg) |
|---|---|---|---|
| Ex.25 | trimethylamine | 165.9 | 56.2 |
| Ex.26 | triethylamine | 190.5 | 77.0 |
| Ex.27 | tri-n-propylamine | 27.2 | 13.4 |
| Ex.28 | tri-n-butylamine | 55.4 | 29.3 |
| Ex.29 | triallylamine | 4.9 | 3.1 |
| Ex.30 | N-methylmorpholine | 5.1 | 2.5 |
| Ex.31 | N,N-dimethyl-cyclohexylamine | 114.2 | 45.1 |
| Ex.32 | N,N,N',N'-tetramethyl-thylenediamine | 50.6 | 19.5 |
| Ex.33 | tetramethylammonium hydroxide | 192.1 | 65.7 |
| Ex.34 | tetraethylammonium hydroxide | 182.4 | 69.5 |
| Ex.35 | cetyltrimethyl hydroxide | 183.1 | 60.4 |
| Ex.36 | trioctylmethyl hydroxide | 167.1 | 61.2 |
| Ex.37 | tetra-n-butyl hydroxide | 174.6 | 62.6 |
| Ex.38 | benzyltriethyl hydroxide | 181.6 | 69.0 |
| Ex.39 | tetradecyldimethylbenzyl hydroxide | 170.7 | 61.0 |
| Ex.40 | trimethylphenyl hydroxide | 151.8 | 57.9 |
| Ex.41 | benzyldimethylphenyl hydroxide | 135.3 | 50.0 |
| Ex.42 | ammonia | 99.9 | 42.8 |
| Ref.Ex.2 | - | 0 | 0 |

Example 43 to 48

There was dissolved 40.8 mg of t-EBA in 2.0 ml of a solution of ethanol and water in a volume ratio of 3 : 1 and then 48.4 mg of L-Lys (Tfa) was added. And to the suspension 0.2 ml of a solution containing alkali metal hydroxide (0.2 mmol) shown in Table 2 was added rapidly at 20°C or while cooling with ice at 0°C and was stirred for 30 minutes. The product was analyzed in the same manner as in Example 1 and the formation of $N^2$-(1-ethoxycarbonyl-3-oxo-3phenylpropyl)-$N^6$-trifluoroacetyl-L-lisine was found.

The amounts of the obtained (S, S) form and (R, S) form are shown in Table 3.

Table 3

| Example No. | alkali metal | reaction temprature ($^{o}C$) | amound of the (S,S) form (mg) | amount of the (R,S) form (mg) |
|---|---|---|---|---|
| Ex.43 | Li | 0 | 43.6 | 10.9 |
| Ex.44 | | 20 | 37.6 | 11.9 |
| Ex.45 | Na | 0 | 37.2 | 11.9 |
| Ex.46 | | 20 | 37.3 | 15.3 |
| Ex.47 | K | 0 | 39.8 | 12.8 |
| Ex.48 | | 20 | 36.7 | 15.0 |

Example 49

After 2.45 g of t-EBA was dissolved in 50.0.ml of a solution of ethanol and water in a volume ratio of 3 : 1 and 2.42 g of L-Lys (Tfa) was suspended, the reaction mixture was warmed to 30°C. And thereto 2.45 g of potassium carbonate was added rapidly and the reaction mixture was stirred. The stirring was continued for 60 minutes. It took 20 minutes to make the reaction mixture almost homogeneous. After completion of the stirring, the reaction was stopped by adding 1.67 ml of concentrated hydrochloric acid. The reaction mixture was analyzed by HPLC and the formation of 3.48 g of $N^2$-(1-ethoxycarbonyl-3-oxo-3-phenylpropyl)-$N^6$-trifluoroacetyl-L-lysine was found. The ratio of the produced diastereomer of the (S, S) form the (R, S) form was 61.0/39.0.

After the solvent of this reaction mixture was distilled away under reduced pressure, 50 ml of ethanol was added to the obtained residue. And thereto 4.0 ml of 2.5N NaOH was gradually added by stirring while cooling with ice at 0°C and then the solvent was distilled away under reduced pressure at a room temperature. After the residue was extracted with ethyl acetate and filtered, the extracted solution was washed, dried with sodium sulfate, concentrated under reduced pressure, and crystalized by adding ether-n-hexane to it.

Consequently, 3.08 g of $N^2$-(1-ethoxycarbonyl-3-oxo-3-phenylpropyl)-$N^6$-trifluoroacetyl-L-lysine was obtained. The ratio of the (S, S) form/the (R, S) form was 60.5/39.5.

Example 50

After 81.6 g of t-EBA was dissolved in 1.0ℓ of a solution of ethanol and water in a volume ratio of 3 : 1 and 48.4 g of L-Lys (Tfa) was suspended, the reaction mixture was cooled with ice to 3°C. There was added dropwise 200.0 ml of a solution of 1N-LiOH over 20 minutes while stirring the mixture and the stirring was continued for additional 40 minutes after completion of the dropping. The reaction was stopped by adding 33.3 ml of concentrated hydrochloric acid and the reaction mixture was analyzed by HPLC. The formation of 84.9 g of $N^2$-(1-ethoxycarbonyl-3-oxo-3-phenylpropyl)-$N^6$-trifluoroacetyl-L-lysine was found. The ratio of the (S, S) form/the (R, S) form was 78.0/22.0.

The obtained reaction mixture was treated in the same manner as in Example 49 and 75.8 g of $N^2$-(1-ethoxycarbonyl-3-oxo-3-phenylpropyl)-$N^6$-trifluoroacetyl-L-lysine was obtained in the form of crystals. The ratio of the (S, S) form/the (R, S) form was 79.0/21.0.

The obtained product had the following properties.

$^1$H-NMR(90 MHz, $CDC1_3$+DMSO-$d_6$) : δ(ppm) ; 1.15 to 1.4(t, 3H), 0.9 to 1.9(m, 6H), 3.0 to 3.55(m, 5H), 3.6 to 3.85 (m, 1H), 3.95 to 4.3 (q, 2H), 7.3 to 8.1 (m, 5H), 8.13 to 8.53 (m, 1H)

IR($cm^{-1}$, KBr disk) : 3375, 2950, 1710, 1630, 1600, 1550, 1210, 1185, 690

$[\alpha]_D^{25}$ =+23.7° (c = 1.0, N-HCℓ)

Example 51

After 40.8 g of t-EBA was dissolved in 500 ml of ethanol and 48.4 g of L-Lys (Tfa) was suspended, the reaction mixture was cooled to –5°C. And thereto 200.0 ml of a solution of IN-LiOH was continuously dropped over 150 minutes while stirring the mixture and the stirring was continued for additional 30 minutes after the dropping was over. The internal temperature of reaction mixture was kept at –5°C during the reaction.

After the reaction was stopped by adding 33.3 ml of concentrated hydrochloric acid, the reaction mixture was analyzed by HPLC and the formation of 81.4 g of $N^2$-(1-ethoxycarbonyl-3-oxo-3-phenylpropyl)-$N^6$-trifluoroacetyl-L-lysine was found. The ratio of the (S, S) form/the (R, S) form was 82.1/17.9.

The obtained reaction mixture was treated in the same manner as in Example 49 and 65.0 g of $N^2$-(1-ethoxycarbonyl-3-oxo-3-phenylpropyl)-$N^6$-trifluoroacetyl-L-lysine was obtained in the form of crystals. The ratio of the (S, S) form/the (R, S) form was 81.9/18.1.

Example 52

After 13.1 ml of concentrated hydrochloric acid was added to 500 ml of ethanol and then 35.0 g of $N^2$-(1-ethoxycarbonyl-3-oxo-3-phenylpropyl)-$N^6$-trifluoroacetylL-lysine obtained in Example 51 was dissolved in this solvent, the hydrogenation was carried out at 40°C under the atmospheric pressure by adding 10.5 g of 10% palladium/carbon. After the reaction was over, the catalyst was removed by suction filtration and the pH of this ethanol solution was adjusted to 4.5 with NaOH. The water was added thereto and ethanol was distilled away by evaporating under reduced pressure resulting in substitution of ethanol with water.

And there was obtained 32.0 g of $N^2$-(1-ethoxycarbonyl-3-phenyl-propyl)-$N^6$-trifluoroacetyl-L-lysine by filtration of the precipitated white crystals. The ratio of the (S, S) form/the (R, S) form was 81.9/18.1. By recrystalizing this product from water-ethanol, $N^2$-(1-(S)-ethoxycarbonyl-3-phenylpropyl)-$N^6$-trifluoroacetyl-L-lysine was obtained.

The obtained product had the following properties.

$^1$H-NMR(90 MHz, $CDCl_3$) : δ(ppm) ; 1.2 to 1.43(t, 3H), 1.42 to 2.25(m, 8H), 2.5 to 2.85(m, 2H), 3.0 to 3.55(m, 4H), 4.05 to 4,35(q, 2H), 6.9 to 7.4(m, 5H)

IR($cm^{-1}$, KBr disk) : 3320, 1740, 1700, 1615, 1205, 1170, 750, 700

mp : 135.5 to 137.0°C

$[\alpha]_D^{25}$ =+7,8°(c = 2,0, EtOH)

Example 53

After 8.16 g of t-EBA was dissolved in 100 ml of ethanol and then 9.69 g of L-Lys (Tfa) was suspended, 20.0 ml of a solution of 1N-LiOH was continuously added dropwise over 150 minutes while stirring the mixture and the stirring was continued for additional 30 minutes after the adding was over. After the reaction was stopped by adding 10.0 ml of concentrated hydrochloric acid, the hydrogenation was carried out at 40°C under the atmospheric pressure by adding 5.0 g of 10% palladium/carbon.

The catalyst was removed at the end of the reaction and 14.1 g of $N^2$-(1-ethoxycarbonyl-3-phenylpropyl)-$N^6$-trifluoroacetyl-L-lysine was obtained after treating the mixture in the same manner as in Example 52. The ratio of the (S, S) form/the (R, S) form was 82.0/18.0.

In addition to the ingredients used in the Examples, other ingredients can be used in the Examples as set forth in the specification to obtain substantially the same results.

## Claims

1. A process for preparing an $N^2$-(1-carboxy-3-oxo-3-phenylpropyl)-L-lysine derivative having the formula (I) :

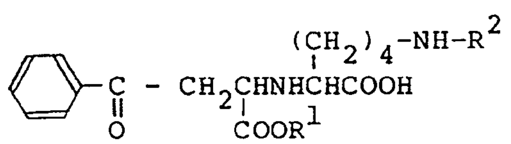

(I)

wherein $R^1$ is an alkyl group and $R^2$ is an acyl or urethane type protecting group which is stable on catalytic hydrogenolysis, which comprises reacting a β-benzoylacrylic acid ester having the formula (II) :

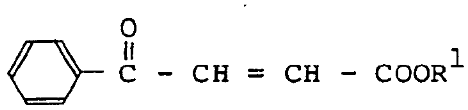

(II)

wherein $R^1$ is as defined above, with an L-lysine derivative having the formula (III) :

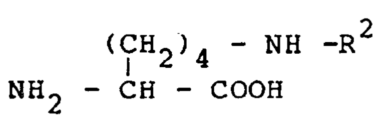

(III)

wherein $R^2$ is as defined above, in the presence of a base in an amount equivalent to the compound (III).

2. The process of Claim 1, wherein the base is a member selected from the group consisting of an alkali metal hydroxide or carbonate, an alkaline earth metal hydroxide, a quaternary ammonium hydroxide, ammonia and an amine.

3. The process of Claim 1, wherein the L-lysine derivative (III) is $N^6$-trifluoroacetyl-L-lysine and the β-benzoylacrylic acid ester (II) is trans-ethyl β-benzoylacrylate.

4. The process of Claim 3, wherein an $N^6$-trifluoroacetyl-L-lysine is reacted with trans-ethyl β-benzoylacrylate in the presence of an alkali metal hydroxide selected from the group consisting of lithium hydroxide, sodium hydroxide and potassium hydroxide to predominantly give $N^2$-[1-(S)-ethoxycarbonyl-3-oxo-3-phenylpropyl]-$N^6$-trifluoroacetyl-L-lysine.

5. The process of Claim 1, wherein after completion of the reaction, a mineral acid is added to the reaction mixture in an amount of not less than the equivalent to the base to stabilize the product.

6. A process for preparing an $N^2$-(1-carboxy-3-phenylpropyl)-L-lysine derivative having the formula (IV) :

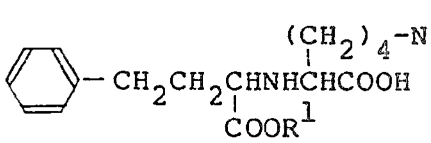
(IV)

wherein $R^1$ is an alkyl group and $R^2$ is an acyl or urethane type protecting group which is stable on catalytic hydrogenolysis, which comprises reacting a β-benzoylacrylic acid ester having the formula (II) :

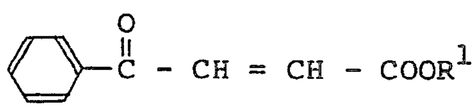
(II)

wherein $R^1$ is as defined above, with an L-lysine derivative having the formula (III) :

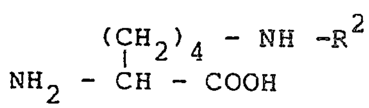
(III)

wherein $R^2$ is as defined above, in the presence of a base in an amount equivalent to the compound (III), to prepare an $N^2$-(1-carboxy-3-oxo-3-phenylpropyl)-L-lysine derivative having the formula (I) :

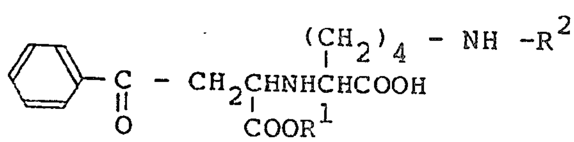
(I)

wherein $R^1$ and $R^2$ as defined above, and conducting catalytic hydrogenolysis of the compound (I).

7. The process of Claim 6, wherein the $N^2$-(1-carboxy-3-oxo-3-phenylpropyl)-L-lysine derivative (I) is $N^2$-(1-ethoxycarbonyl-3-oxo-3-phenylpropyl)-$N^6$-trifluoroacetyl-L-lysine which is catalytically hydrogenolyzed to produce $N^2$-(1-ethoxycarbonyl-3-phenylpropyl)-$N^6$-trifluoroacetyl-L-lysine.

8. An $N^2$-(1-carboxy-3-phenylpropyl)-L-lysine derivative having the formula (XII) :

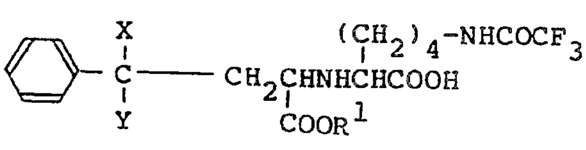
(XII)

wherein $R^1$ is an alkyl group, and each of X and Y is hydrogen atom or X and Y are taken together to form oxygen atom.

9. The derivative of Claim 8, wherein $N^2$-(1-ethoxycarbonyl-3-phenylpropyl)-$N^6$-trifluoroacetyl-L-lysine has (S)-configuration with respect to the asymmetric carbon atom at the 1-position of the propyl group.

## Ansprüche

1. Verfahren zur Herstellung eines $N^2$-(1-Carboxy-3-oxo-3-phenylpropyl)-L-lysinderivats der Formel (I) :

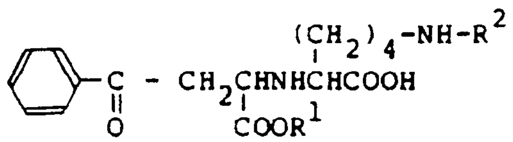

(I)

wobei $R^1$ eine Alkylgruppe ist und $R^2$ eine Schutzgruppe vom Acyl- oder Urethantyp ist, die gegenüber katalytischer Hydrierung beständig ist, das die Reaktion eines β-Benzoylacrylsäureesters der Formel (II) umfaßt :

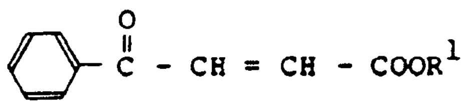

(II)

wobei $R^1$ wie oben definiert ist, mit einem L-Lysinderivat der Formel (III) :

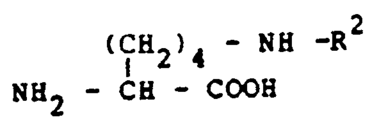

(III)

wobei $R^2$ wie oben definiert ist, in Gegenwart einer zur Verbindung (III) äquimolaren Menge einer Base.

2. Verfahren nach Anspruch 1, wobei die Base ein Mitglied ausgewählt aus der Gruppe ist, die aus einem Alkalimetallhydroxid oder -carbonat, einem Erdalkalimetallhydroxid, einem quarternären Ammoniumhydroxid, Ammoniak und einem Amin besteht.

3. Verfahren nach Anspruch 1, wobei das L-Lysinderivat (III) $N^6$-Trifluoroacetyl-L-lysin ist und der β-Benzoylacrylsäureester (II) trans-Ethyl β-Benzoylacrylat ist.

4. Verfahren von Anspruch 3, wobei ein $N^6$-Trifluoroacetyl-L-lysin mit transEthyl β-Benzoylacrylat umgesetzt wird in Gegenwart eines Alkalimetallhydroxid ausgewählt aus der Gruppe, die aus Lithiumhydroxid, Natriumhydroxid und Kaliumhydroxid besteht, um überwiegend $N^2$-[1-(S)-Ethoxycarbonyl-3-oxo-phenylpropyl]-$N^6$-trifluoroacetyl-L-lysin zu erhalten.

5. Verfahren von Anspruch 1, wobei nach Vervollständigung der Reaktion zur Reaktionsmischung eine Mineralsäure gegeben wird, in einer nicht geringeren Menge als das Äquivalent zur Base zur Stabilisierung des Produkts.

6. Verfahren zur Herstellung eines $N^2$-(1-Carboxy-3-phenylpropyl)-L-lysinderivats der Formel (IV) :

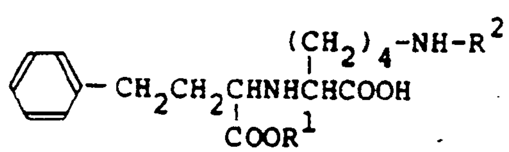

(IV)

wobei $R^1$ eine Alkylgruppe ist und $R^2$ eine Schutzgruppe von Acyl- oder Urethantyp ist, die gegenüber katalytischer Hydrierung beständig ist, das die Reaktion eines β-Benzoylacrylsäureesters der Formel (II) umfaßt :

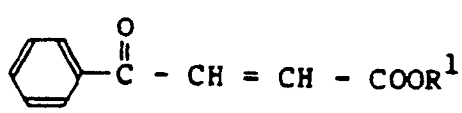

(II)

wobei $R^1$ wie oben definiert ist, mit einem L-Lysinderivat der Formel (III) :

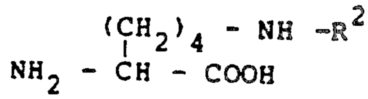

(III)

wobei $R^2$ wie oben definiert ist, in Gegenwart einer zur Verbindung (III) äquimolaren Menge einer Base, um ein $N^2$-(1-Carboxy-3-oxo-3-phenylpropyl)-L-lysinderivat der Formel (I) herzustellen :

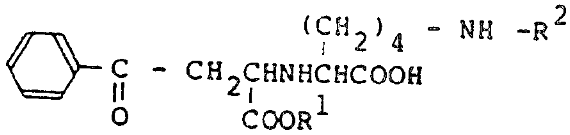

(I)

wobei $R^1$ und $R^2$ wie oben definiert sind, und Durchführung der katalytischen Hydrierung der Verbindung (I).

7. Verfahren nach Anspruch 6, wobei das $N^2$-(1-Carboxy-3-oxo-3-phenylpropyl)-L-lysinderivat (I), $N^2$-(1-Ethoxycarbonyl-3-oxo-3-phenylpropyl)-$N^6$-trifluoroacetyl-L-lysin ist, welches katalytisch hydriert wird, um $N^2$-(1-Ethoxycarbonyl-3-phenylpropyl)-$N^6$-trifluoroacetyl-L-lysin herzustellen.

8. Ein $N^2$-(1-Carboxy-3-phenylpropyl)-L-lysinderivat der Formel (XII) :

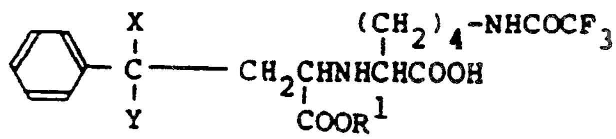

(XII)

wobei $R^1$ eine Alkylgruppe ist, und X und Y jeweils ein Wasserstoffatom sind, oder X und Y zusammen ein Sauerstoffatom bilden.

9. Derivat von Anspruch 8, wobei $N^2$-(1-Ethoxycarbonyl-3-phenylpropyl)-$N^6$-trifluoroacetyl-L-lysin hinsichtlich des asymmetrischen Kohlenstoffatoms an der 1-Position der Propylgruppe (S)-Konfiguration hat.

## Revendications

1. Procédé pour préparer un dérivé du $N^2$ (carboxy-1 oxo-3 phényl-3 propyl) L-lysine répondant à la formule (I) :

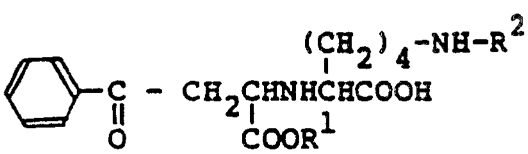

(I)

dans laquelle, $R^1$ réprésente un groupement alkyle et $R^2$ représente un groupement de protection de type acyle ou uréthane qui est stable à l'hydrogénolyse catalytique, qui comprend la réaction d'un ester de l'acide benzoylacrylic de formule (II) :

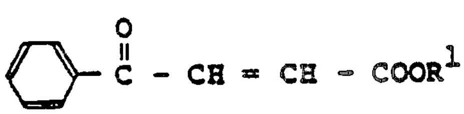

(II)

pour lequel $R^1$ a la même signification que précédemment, avec un dérivé de la L-lysine de formule (III) :

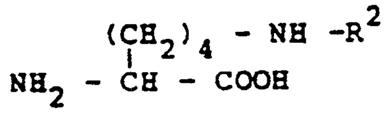

(III)

pour lequel R² a la même signification que précédemment, en présence d'une base en quantité équivalente au composé (III).

2. Procédé selon la revendication 1, pour lequel la base est un membre sélectionné parmi le groupe des hydroxides ou carbonates de métal alcalin, des hydroxides de métal alcalino-terreux, des hydroxides d'ammoniums quaternaires, de l'ammoniac et d'une amine.

3. Procédé selon la revendication 1, pour lequel le dérivé de la L-lysine (III) est la $N^6$-trifluoroacetyl L-lysine et l'ester de l'acide benzoylacrylic (II) est le trans-éthyl β-benzoyle acrylate.

4. Procédé selon la revendication 3, pour lequel on fait réagir de la $N^6$-trifluoroacetyl L-lysine avec le trans-éthyl -benzolacrylate en présence d'hydroxide de métal alcalin sélectionné parmi le groupe des hydroxide de lithium, hydroxide de sodium et hydroxide de potassium pour donner principalement le $N^2$- [(S) éthoxycarbonyl-1 oxo-3 phényl-3 propyl] $N^6$-trifluoroacétyl L-lysine.

5. Procédé selon la revendication 1, pour lequel on ajoute, lorsque la réaction est terminée, un acide minéral au mélange réactionnel en une quantité qui n'est pas inférieure à l'équivalent en base, pour stabiliser le produit.

6. Procédé pour préparer un dérivé du $N^2$-(carboxy-1 phényl-3 propyl) L-lysine répondant à la formule (IV):

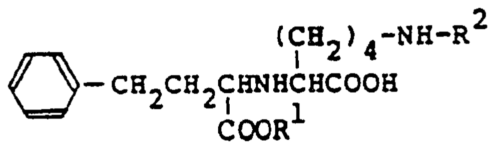

(IV)

dans laquelle R¹ représente un groupement alkyle et R² représente un groupement de protection de type acyle ou uréthane qui est stable à l'hydrogénolyse catalytique, qui comprend la réaction d'un ester de l'acide benzoylacrylique de formule (II) :

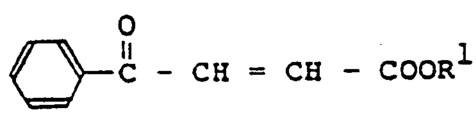

(II)

pour lequel R¹ a la même signification que précédemment, avec un dérivé de la L-lysine de formule (III) :

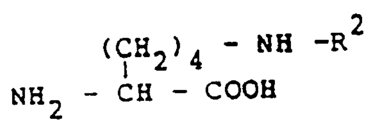

(III)

pour lequel R² à la même signification que précédemment, en présence d'une base en quantité équivalente au composé (III), pour préparer un dérivé du $N^2$-(carboxy-1 oxo-3 phényl-3 propyl) L-lysine de formule (I) :

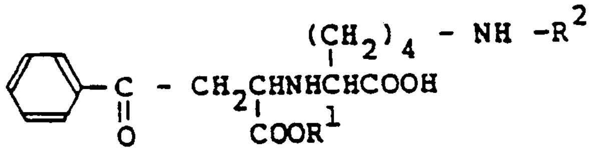

(I)

pour lequel R¹ et R² ont la même signification que précédemment, et l'hydrogénolyse catalytique du composé (I).

7. Procédé selon la revendication 6, pour lequel le dérivé du $N^2$-(carboxy- 1 oxo-3 phenyl-3 propyl) L-lysine est le $N^2$-(ethoxycarbonyl- 1 oxo-3 phényl-3 propyl) $N^6$-trifluoroacéthyl L-lysine qui est hydrogénolysé catalytiquement pour produire $N^2$-(-ethoxycarbonyl-1 phényl-3) $N^6$-trifluoroacétyl L-lysine.

8. Dérivé du N²-(carboxy-1 phényl-3 propyl) L-lysine répondant à la formule (XII) :

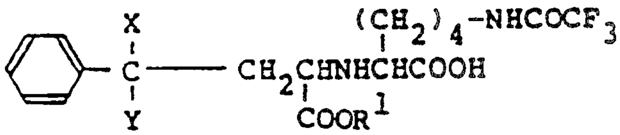

(XII)

Pour lequel $R^1$ est un groupement alkyl, et chacun des substituants X et Y représente un atome d'hydrogène ou X et Y sont assemblés pour former un atome d'oxygène.

9. Dérivé selon la revendication 8, pour lequel le $N^2$-(éthoxycarbonyl- 3phény1 propyl) $N^6$ -trifluoroacétyl L-lysine à la configuration (S) quant au carbone asymétrique de la position-1 du groupement propyle.